# EUROPEAN PATENT APPLICATION

(11) **EP 4 180 511 A1**
(43) Date of publication of application: **17.05.2023**
(21) Application number: 21837468.4
(22) Date of filing: 31.05.2021
(51) Int. Cl.: C12M 1/00, C12N 1/00

(54) **CELL CULTURING SYSTEM**

(30) Priority: 07.07.2020 JP 2020116949
(71) Applicant: SHIMADZU CORPORATION, Kyoto-shi, Kyoto 604-8511 (JP)
(72) Inventor: YONEDA, Yasuko, Kyoto-shi, Kyoto 604-8511 (JP); OHKUBO, Tomoki, Kyoto-shi, Kyoto 604-8511 (JP); HASHIMOTO, Toyoyuki, Kyoto-shi, Kyoto 604-8511 (JP)
(74) Representative: Kilian Kilian & Partner
(86) International application number: PCT/JP2021/020605
(87) International publication number: WO 2022/009560

(57) **Abstract**

A cell culture system (100) includes: a cell culture container (10) disposed in an anaerobic chamber (70); and an external apparatus (30, 40, 50, 60). The external apparatus (30, 40, 50, 60) has a main body unit (30, 40, 50) disposed in the anaerobic chamber (70) and an operation unit (60) disposed outside the anaerobic chamber (70). The main body unit (30, 40, 50) is configured to perform a predetermined process onto the cell culture container (10). The operation unit (60) is configured to transmit or receive a signal associated with the predetermined process to or from the main body unit (30, 40, 50) by wireless communication.

## Description

### TECHNICAL FIELD

The present invention relates to a cell culture system.

### BACKGROUND ART

PTL 1 (WO 2018/079793) discloses a system in which a device having intestinal epithelial cells seeded on a porous membrane is disposed in an anaerobic chamber and the intestinal epithelial cells and bacteria included in a culture medium are co-cultured.

### CITATION LIST

### PATENT LITERATURE

PTL 1: WO 2018/079793

### SUMMARY OF INVENTION

### TECHNICAL PROBLEM

In order to perform circulation of the culture medium, measurement of an electrical resistance value of the intestinal epithelial cells on the porous membrane, measurement of a dissolved oxygen concentration of the culture medium, and the like in the system of PTL 1, an external apparatus necessary for each of these processes is also disposed in the anaerobic chamber. Since the external apparatus is operated by inserting a hand into the anaerobic chamber from a glove or sleeve provided at a front surface of the anaerobic chamber, operability is low.

In order to improve the operability, it is considered to divide the external apparatus into a main body unit disposed in the anaerobic chamber and an operation unit disposed outside the anaerobic chamber and to connect the main body unit and the operation unit together via a cable. In this case, the operability of the external apparatus is improved; however, since the anaerobic chamber is provided with a through hole through which the cable extends, another measure is required to ensure airtightness of the anaerobic chamber around the through hole.

The present invention provides a cell culture system to improve operability of an external apparatus without providing an anaerobic chamber with a through hole.

### SOLUTION TO PROBLEM

A cell culture system of the present invention includes: a cell culture container disposed in an anaerobic chamber; and an external apparatus. The external apparatus has a main body unit disposed in the anaerobic chamber and an operation unit disposed outside the anaerobic chamber. The main body unit is configured to perform a predetermined process onto the cell culture container. The operation unit is configured to transmit or receive a signal associated with the predetermined process to or from the main body unit by wireless communication.

### ADVANTAGEOUS EFFECTS OF INVENTION

According to the cell culture system of the present invention, operability of the external apparatus can be improved without providing the anaerobic chamber with a through hole.

### BRIEF DESCRIPTION OF DRAWINGS

Fig. 1 is a schematic diagram of a cell culture system 100.
Fig. 2 is a block diagram of a pump 30.
Fig. 3 is a block diagram of an electrical resistance measurement apparatus 40.
Fig. 4 is a block diagram of a dissolved-oxygen concentration measurement apparatus 50.

### DESCRIPTION OF EMBODIMENTS

Embodiments of the present invention will be described with reference to figures. Here, the same or corresponding portions are denoted by the same reference characters, and the same explanation will not be described repeatedly.

### (Configuration of Cell Culture System according to Embodiment)

Hereinafter, a configuration of a cell culture system (hereinafter, referred to as "cell culture system 100") according to an embodiment will be described.

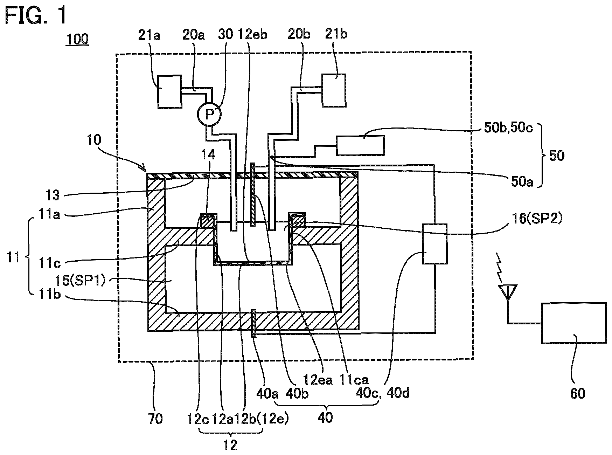

Fig. 1 is a schematic diagram of cell culture system 100. As shown in Fig. 1, cell culture system 100 includes a cell culture container 10, tubes 20a and 20b, a pump 30, an electrical resistance measurement apparatus 40, a dissolved-oxygen concentration measurement apparatus 50, an operation unit 60, and an anaerobic chamber 70. Pump 30, electrical resistance measurement apparatus 40, dissolved-oxygen concentration measurement apparatus 50, and operation unit 60 are each an external apparatus of cell culture system 100. Pump 30, electrical resistance measurement apparatus 40, and dissolved-oxygen concentration measurement apparatus 50 are each a main body unit of the external apparatus.

### <Cell Culture Container 10>

Cell culture container 10 has a container main body 11, a cell culture insert 12, a cover member 13, and a sealing member 14.

Container main body 11 has a side wall 11a, a bottom wall 11b, and a partition wall 11c. Bottom wall 11b closes the lower end side of side wall 11a. The upper end side of side wall 1 1a is opened. This opening is closed by cover member 13. Partition wall 11c is contiguous to side wall 11a between the upper end of side wall 11a and the lower end of side wall 11a. Partition wall 11c extends in parallel with bottom wall 11b, for example. An opening 11ca is formed in partition wall 11c. Opening 11ca extends through partition wall 11c along its thickness direction.

Cell culture insert 12 has a tubular portion 12a, a bottom portion 12b, and a flange portion 12c. Tubular portion 12a has a tubular shape. The upper end of tubular portion 12a is opened. The lower end of tubular portion 12a is closed by bottom portion 12b.

Bottom portion 12b is a membrane 12e. Membrane 12e is oxygen-permeable. Membrane 12e is, for example, a track-etched membrane composed of polycarbonate. Membrane 12e has a first main surface 12ea and a second main surface 12eb. Second main surface 12eb is opposite to first main surface 12ea.

Tubular portion 12a is inserted into opening 11ca such that bottom portion 12b (membrane 12e) is located between bottom wall 11b and partition wall 11c. A first space SP1 represents a space defined by side wall 11a between bottom wall 11b and partition wall 11c, bottom wall 11b, partition wall 11c, an outer peripheral surface of tubular portion 12a located below partition wall 11c, and membrane 12e. A second space SP2 represents an inner space of tubular portion 12a.

First main surface 12ea is a main surface of membrane 12e on the first space SP1 side, and second main surface 12eb is a main surface of membrane 12e on the second space SP2 side. That is, first space SP1 is partially defined by first main surface 12ea, and second space SP2 is partially defined by second main surface 12eb.

A first culture medium 15 and a second culture medium 16 are stored in first space SP1 and second space SP2, respectively. Second culture medium 16 is a culture medium having a dissolved oxygen concentration lower than a dissolved oxygen concentration of first culture medium 15. Second culture medium 16 includes, for example, anaerobic bacteria. Cells are cultured on second main surface 12eb. These cells are, for example, intestinal epithelial cells that form a tight junction on second main surface 12eb. Specific examples of such cells include Caco-2 cells. Oxygen in first culture medium 15 is supplied to the cells through membrane 12e.

Flange portion 12c protrudes from the outer peripheral surface of tubular portion 12a in a plane orthogonal to the extending direction of tubular portion 12a. Flange portion 12c is located at the upper end of tubular portion 12a. Sealing member 14 seals between flange portion 12c and partition wall 11c in an airtight manner.

### <Tubes 20a and 20b>

Tube 20a extends through cover member 13. Tube 20a has one end connected to second space SP2. Tube 20a has the other end connected to a culture medium container 21a. Second culture medium 16 is stored in culture medium container 21a.

Tube 20b extends through cover member 13. Tube 20b has one end connected to second space SP2. Tube 20b has the other end connected to a culture medium container 21b. Second culture medium 16 used for cell culturing in second space SP2 is stored in culture medium container 21b.

### <Pump 30>

Pump 30 is, for example, a tube pump. However, pump 30 is not limited thereto. Pump 30 delivers second culture medium 16 stored in culture medium container 21a to second space SP2 via tube 20a, and discharges second culture medium 16 stored in second space SP2 to culture medium container 21b via tube 20b. In this way, replacement of second culture medium 16 is performed.

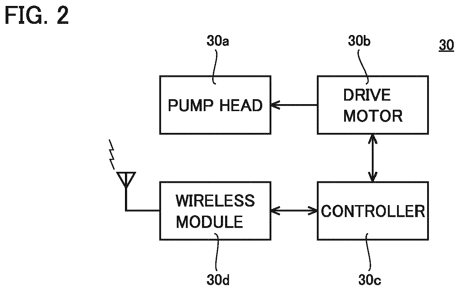

Fig. 2 is a block diagram of pump 30. As shown in Fig. 2, pump 30 has a pump head 30a, a drive motor 30b, a controller 30c, and a wireless module 30d.

Pump head 30a is attached to tube 20a and is driven to rotate by drive motor 30b so as to perform the above-described delivering operation or the like.

Controller 30c is constituted of, for example, a microcontroller. Controller 30c controls a rotation speed of drive motor 30b or the like based on a control signal supplied from wireless module 30d. Wireless module 30d is a module having a circuit that performs a baseband signal process, a circuit that performs an RF signal process, and an antenna that transmits or receives an RF signal, and configured to perform wireless communication in compliance with WiFi (registered trademark). Wireless module 30d may be configured to perform wireless communication in compliance with Bluetooth (registered trademark). Wireless module 30d receives the above-described control signal.

### <Electrical Resistance Measurement Apparatus 40>

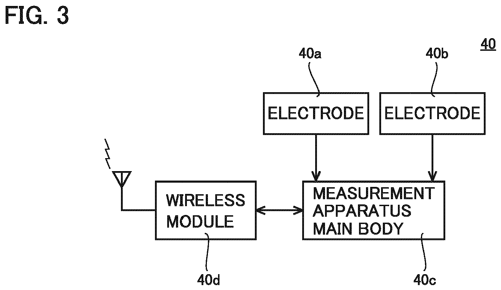

Fig. 3 is a block diagram of electrical resistance measurement apparatus 40. As shown in Fig. 3, electrical resistance measurement apparatus 40 has an electrode 40a, an electrode 40b, a measurement apparatus main body 40c, and a wireless module 40d. Electrode 40a extends through container main body 11 and is in contact with first culture medium 15 (see Fig. 1). Electrode 40b extends through cover member 13 and is in contact with second culture medium 16 (see Fig. 1).

Measurement apparatus main body 40c is a trans-epithelial electrical resistance (TEER) measurement apparatus. Measurement apparatus main body 40c is connected to electrodes 40a and 40b. Measurement apparatus main body 40c measures an electrical resistance value between electrodes 40a and 40b and outputs the electrical resistance value to wireless module 40d. Wireless module 40d has the same configuration as that of wireless module 30d. Wireless module 40d transmits the above-described electrical resistance value. It should be noted that the electrical resistance value between electrodes 40a and 40b is varied between a case where the cells cultured on second main surface 12eb form a tight junction and a case where the cells cultured on second main surface 12eb do not form a tight junction. Therefore, by the measurement of the electrical resistance value, it is possible to determine whether or not the cells cultured on second main surface 12eb form a tight junction.

### <Dissolved-Oxygen Concentration Measurement Apparatus 50>

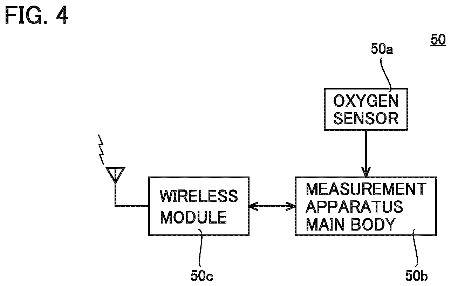

Fig. 4 is a block diagram of dissolved-oxygen concentration measurement apparatus 50. As shown in Fig. 4, dissolved-oxygen concentration measurement apparatus 50 has an oxygen sensor 50a, a measurement apparatus main body 50b, and a wireless module 50c. Oxygen sensor 50a is disposed, for example, on a path of tube 20b (see Fig. 1). Thus, oxygen sensor 50a outputs a signal corresponding to the dissolved oxygen concentration of second culture medium 16 discharged from second space SP2.

Measurement apparatus main body 50b is connected to oxygen sensor 50a. Measurement apparatus main body 50b calculates the dissolved oxygen concentration of second culture medium 16 based on the signal from oxygen sensor 50a, and outputs the dissolved oxygen concentration to wireless module 50c. Wireless module 50c has the same configuration as that of wireless module 30d (wireless module 40d). Wireless module 50c transmits the above-described dissolved oxygen concentration.

### <Operation Unit 60>

Operation unit 60 is constituted of, for example, a personal computer. Operation unit 60 may be constituted of a tablet terminal or like. Operation unit 60 includes the same wireless module as wireless module 30d (wireless module 40d or wireless module 50c).

Operation unit 60 receives an operation for pump 30 via a keyboard, a mouse, a touch panel type liquid crystal display, or the like. Operation unit 60 generates a control signal that is based on this operation and transmits the control signal to wireless module 30d via a wireless module included in operation unit 60. In this way, pump 30 is operated.

The electrical resistance value transmitted from wireless module 40d and the dissolved oxygen concentration transmitted from wireless module 50c are received by the wireless module included in operation unit 60. The received electrical resistance value and dissolved oxygen concentration are stored in a storage device of operation unit 60 or are displayed on the liquid crystal display or the like of operation unit 60.

### <Anaerobic Chamber 70>

Cell culture container 10, tube 20a, tube 20b, culture medium container 21a, culture medium container 21b, pump 30, electrical resistance measurement apparatus 40, and dissolved-oxygen concentration measurement apparatus 50 are disposed in anaerobic chamber 70. Operation unit 60 is disposed outside anaerobic chamber 70.

In the above-described example, replacement of second culture medium 16 is performed; however, replacement of first culture medium 15 may be further performed. Further, in the above-described example, the dissolved oxygen concentration of second culture medium 16 is measured; however, the dissolved oxygen concentration of first culture medium 15 may be further measured.

### (Effects of Cell Culture System according to Embodiment)

Effects of cell culture system 100 will be described below.

In cell culture system 100, the transmission and reception of the signals associated with the processes on cell culture container 10 (the transmission of the control signal to pump 30, the reception of the electrical resistance value from electrical resistance measurement apparatus 40, and the reception of the dissolved oxygen concentration from dissolved-oxygen concentration measurement apparatus 50) can be performed by wireless communication with operation unit 60.

Therefore, in cell culture system 100, operability of each of pump 30, electrical resistance measurement apparatus 40, and dissolved-oxygen concentration measurement apparatus 50 can be improved without providing anaerobic chamber 70 with a through hole through which a cable extends to connect these apparatuses and operation unit 60 together.

When each of pump 30, electrical resistance measurement apparatus 40, and dissolved-oxygen concentration measurement apparatus 50 is operated by inserting a hand from a glove or sleeve provided at a front surface of anaerobic chamber 70, pressure fluctuations or vibrations occurring at the time of inserting the hand may affect cell culturing in cell culture container 10. In cell culture system 100, it is not necessary to insert a hand from a glove or sleeve provided at the front surface of anaerobic chamber 70 in order to operate these apparatuses. Therefore, according to cell culture system 100, pressure fluctuations and vibrations resulting from operations on pump 30, electrical resistance measurement apparatus 40, and dissolved-oxygen concentration measurement apparatus 50 can be suppressed and stable cell culturing can be performed.

Although the embodiments of the present invention have been illustrated, the embodiments described above can be modified in various manners. Further, the scope of the present invention is not limited to the above-described embodiments. The scope of the present invention is defined by the terms of the claims, and is intended to include any modifications within the scope and meaning equivalent to the terms of the claims.

### REFERENCE SIGNS LIST

10: cell culture container; 11: container main body; 11a: side wall; 11b: bottom wall; 11c: partition wall; 11ca: opening; 12: cell culture insert; 12a: tubular portion; 12b: bottom portion; 12c: flange portion; 12e: membrane; 12ea: first main surface; 12eb: second main surface; 13: cover member; 14: sealing member; 15: first culture medium; 16: second culture medium; 20a, 20b: tube; 21a, 21b: culture medium container; 30: pump; 30a: pump head; 30b: drive motor; 30c: controller; 30d: wireless module; 40: electrical resistance measurement apparatus; 40a, 40b: electrode; 40c: measurement apparatus main body; 40d: wireless module; 50: dissolved-oxygen concentration measurement apparatus; 50a: oxygen sensor; 50b: measurement apparatus main body; 50c: wireless module; 60: operation unit; 70: anaerobic chamber; 100: cell culture system; SP1: first space; SP2: second space.

## Claims

1. A cell culture system comprising:
a cell culture container disposed in an anaerobic chamber;
an external apparatus having a main body unit disposed in the anaerobic chamber and an operation unit disposed outside the anaerobic chamber, wherein
the main body unit is configured to perform a predetermined process onto the cell culture container, and
the operation unit is configured to transmit or receive a signal associated with the predetermined process to or from the main body unit by wireless communication.

2. The cell culture system according to claim 1, wherein the cell culture container has an oxygen-permeable membrane, a first space, and a second space, the oxygen-permeable membrane being disposed in the cell culture container, the oxygen-permeable membrane including a first main surface and a second main surface opposite to the first main surface, the second main surface being configured for culturing cells, the first space being partially defined by the first main surface and being configured to store a first culture medium, the second space being partially defined by the second main surface and being configured to store a second culture medium having a dissolved oxygen concentration lower than a dissolved oxygen concentration of the first culture medium.

3. The cell culture system according to claim 2, wherein the predetermined process is one of replacement of the first culture medium or the second culture medium, measurement of the dissolved oxygen concentration of the first culture medium or the second culture medium, and measurement of an electrical resistance value of the cells cultured on a main surface of the membrane facing the second space side.

4. The cell culture system according to claim 1, wherein the wireless communication is performed by communication in compliance with WiFi or Bluetooth.

5. A cell culture system comprising:
an anaerobic chamber;
a cell culture container disposed in the anaerobic chamber;
an external apparatus having a main body unit disposed in the anaerobic chamber and an operation unit disposed outside the anaerobic chamber, wherein
the main body unit is configured to perform a predetermined process onto the cell culture container, and
the operation unit is configured to transmit or receive a signal associated with the predetermined process to or from the main body unit by wireless communication.
